Europäisches Patentamt

European Patent Office

Office européen des brevets

(11) Veröffentlichungsnummer : **0 054 653**
**B1**

(12) **EUROPÄISCHE PATENTSCHRIFT**

(45) Veröffentlichungstag der Patentschrift :
**16.04.86**

(51) Int. Cl.⁴ : **A 61 C   1/08**

(21) Anmeldenummer : **81108430.0**

(22) Anmeldetag : **16.10.81**

(54) **Zahnärztliches Handstück.**

(30) Priorität : **22.12.80 DE 3048383**

(43) Veröffentlichungstag der Anmeldung :
**30.06.82 Patentblatt 82/26**

(45) Bekanntmachung des Hinweises auf die Patenterteilung : **16.04.86 Patentblatt 86/16**

(84) Benannte Vertragsstaaten :
**AT CH FR GB IT LI**

(56) Entgegenhaltungen :
**DE-A- 1 541 200**
**DE-B- 2 209 552**
**DE-C-   573 143**
**DIN 69176, Teil 1, Preisgr. 4, August 1977, Seiten 1-3,**
**Beuth Verlag GmbH, Berlin, DE.**
**Die Akte enthält technische Angaben, die nach dem**
**Eingang der Anmeldung eingereicht wurden und die**
**nicht in dieser Patentschrift enthalten sind.**

(73) Patentinhaber : **Siemens Aktiengesellschaft**
**Berlin und München Wittelsbacherplatz 2**
**D-8000 München 2 (DE)**

(72) Erfinder : **Straihammer, Reinhard**
**In der Hollerhecke 10**
**D-6141 Einhausen (DE)**
Erfinder : **Klose, Hans-Jürgen**
**Gleiwitzerstrasse 44**
**D-6944 Hemsbach (DE)**

EP 0 054 653 B1

## Beschreibung

Bei zahnärztlichen Handstücken besteht die Forderung, die Oberfläche im Griffbereich und in den Bereichen, wo mit den Fingern ein Betätigungsglied, z. B. ein Ventil, zu betätigen ist, so zu gestalten, daß sie einerseits ausreichend griffig, andererseits aber auch leicht zu reinigen ist.

Bekannt ist es, die in Frage kommenden Flächen mit feinen Riffelungen oder Rändelungen zu versehen. Eine diesbezügliche Oberflächengestaltung ist jedoch insofern nicht zufriedenstellend, weil sich in den feinen Rillen Schmutz besonders leicht ansammeln kann und die Rillen schlecht zu reinigen sind.

Um diese Nachteile zu vermeiden, ist es weiter bekannt, an der Oberfläche eine Vielzahl von kleinen, am Umfang gleichmäßig verteilt angeordneten muldenförmigen Abflachungen vorzusehen. Damit läßt sich zwar eine relativ gute Griffigkeit im Hinblick auf die Handstückführung bei ausreichender Sauberhaltung der Handstückoberfläche erzielen, doch könnte eine Haftung innerhalb dieser muldenförmigen Abflachungen noch besser sein.

Aufgabe der vorliegenden Erfindung ist es, die Griffigkeit, insbesondere Rutschfestigkeit, der Handstückoberfläche in den betreffenden Bereichen noch weiter zu verbessern, ohne jedoch die Sauberhaltung der Oberfläche zu verschlechtern.

Das gestellte Ziel wird gemäß der Erfindung dadurch gelöst, daß die in Frage kommenden Handstückteile an ihrer Oberfläche mit einer Trägerschicht versehen sind, in die Körner einer Härte größer 2 000 HV mit ausreichender Haftung derart eingebettet sind, daß eine Oberflächenrauhigkeit gebildet wird, die Körnungen für Schleifmittel auf Unterlagen im Bereich von 240 bis 1 200, vorzugsweise einer Körnung der Korngröße 360 entspricht. Die aufgebrachten Körnungen entsprechen einer Korngrößenverteilung nach DIN 69176 (Ausgabe 8/77) bei Verwendung einer Elektrokorund- und Siliziumkarbidkörnung und DIN 848 (Ausgabe 3/80) bei Verwendung einer Diamantkörnung.

Die gemäß der Erfindung vorgeschlagene Oberflächenbeschaffenheit erlaubt es sogar, auf Profilierungen weitgehend zu verzichten. Sie kann sowohl im Bereich des Griffkörpers eines Handstückes als auch an anderer Stelle, an der eine entsprechende Rutschfestigkeit verlangt wird, vorgesehen sein, so z. B. bei durch Druck oder Verdrehen betätigbaren Stellgliedern.

Die Beschichtung ist vorteilhafterweise galvanisch auf die Handstückoberfläche aufgebracht. Als Einlagerungen können vorteilhafterweise Korund- oder Diamantkörner vorgesehen sein. Aus optischen Gründen und auch, um eine gewisse Glättung der Oberfläche zu bekommen, ist es vorteilhaft, die Trägerschicht, in der die Körner eingebettet sind, vorteilhafterweise ist dies eine Nickelschicht, noch mit einer Chromschicht zu überziehen.

Ein Ausführungsbeispiel der Erfindung wird nachfolgend anhand der Zeichnung näher erläutert. Es zeigen:

Figur 1 ein zahnärztliches Handstück in einer perspektivischen Darstellung,

Figur 2 einen sehr kleinen Teil des Handstückes im Längsschnitt in x-facher Vergrößerung.

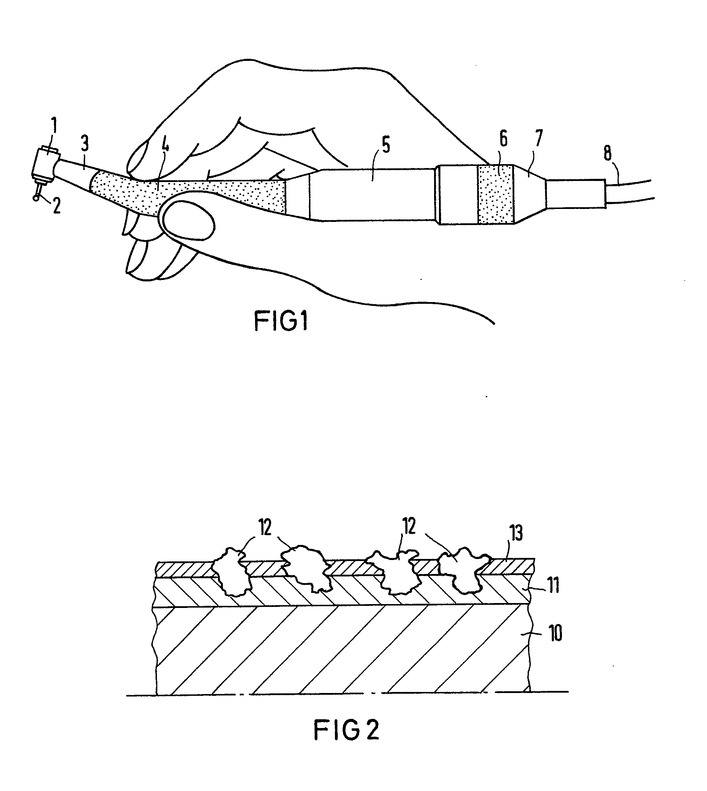

Die Fig. 1 zeigt in einer schaubildlichen Darstellung ein zahnärztliches Handstück mit einem Kopfgehäuse 1 zur Aufnahme eines rotierenden Werkzeuges 2, mit einem sich daran anschließenden Hals- und Griffteil 3, 4 und einem mit 5 bezeichneten Basisteil, in dem ein Antrieb, z. B. ein Elektromotorantrieb, für das Werkzeug 2 angeordnet ist. Mit 6 ist ein Drehring bezeichnet, der zur Betätigung eines im Innern des Handstückes angeordneten, in der Darstellung nicht gezeichneten Mengenregulierventils für zur Kühlung der Präparationsstelle in bekannter Weise vorgesehene Medien Luft und/oder Wasser dient. Das gesamte Handstück ist über eine Anschlußarmatur 7 mit einem Versorgungsschlauch 8 zur Zuführung dieser Medien verbunden.

Der Einstellring 6 sowie der Griffkörperteil 4 weisen eine nachfolgend noch näher erläuterte Oberflächengestaltung auf, die eine äußerst gute Griffigkeit, insbesondere Rutschfestigkeit, bezüglich der Handhabung bietet, wodurch ein sicherer Halt des Handstückes auch bei ungünstigen Bedingungen (z. B. bei feuchten Händen) gegeben ist.

Anhand der Fig. 2 wird die Oberflächengestaltung im Bereich des Griffkörpers 4 bzw. des Einstellringes 6 näher erläutert.

Auf der Oberfläche des Rohlinges des betreffenden Handstückteils, welcher, in der Fig. mit 10 bezeichnet, aus einem galvanisierbaren Material, z. B. Stahl, Messing oder verkupfertem Messing, besteht, ist elektrolytisch eine 0,003 bis 0,015 mm starke Trägerschicht 11 aus Nickel aufgebracht. In diese Trägerschicht 11 sind Körner 12 sehr hoher Härte (größer 2 000 HV) eingebettet, und zwar so weit, daß sie eine ausreichende Haftung haben. Diese wird in der Regel dadurch erreicht, daß man die Körner etwa mit der Hälfte ihres Volumens — und gegebenenfalls mit noch größerem Volumen — in die Trägerschicht 11 einbettet. Die Trägerschicht kann je nach Verfahrensweise ein- oder mehrlagig aufgebracht sein.

Auf die Trägerschicht 11 wiederum ist eine relativ dünne, im Bereich zwischen 0,1 und 3 μm starke Chromschicht 13 aufgebracht, die außer einer optischen Anpassung an benachbarte Handstückteile auch eine gewisse Glättung der Oberfläche bewirkt, und zwar dergestalt, daß die herausragenden Anteile der Körner 12 die geforderte Rauhigkeit erbringen. Die vorteilhafterweise verwendete Körnung liegt nach DIN 69176 für z. B. Korundkörnung im Bereich der Mikrokörnung, also zwischen P 240 und P 1200. Als besonders vorteilhaft hat sich eine Körnung von 360 (P 360 nach DIN 69176) erwiesen.

Werden andere Körner als Korundkörner verwendet, so gelten hinsichtlich der verwendeten Korngrößen entsprechende Festlegungen. Internationale Festlegungen (z. B. ISO-Norm) sind entsprechend anwendbar.

## Patentansprüche

1. Zahnärztliches Handstück, dadurch gekennzeichnet, daß zumindest Teile seiner Oberfläche mit einer Trägerschicht (11) versehen sind, in die Körner (12) einer Härte größer 2 000 HV mit ausreichender Haftung derart eingebettet sind, daß eine Oberflächenrauhigkeit gebildet wird, die Körnungen für Schleifmittel auf Unterlagen im Bereich von 240 bis 1 200, vorzugsweise einer Körnung der Korngröße 360, entspricht, nach DIN 69176 (Ausgabe 8/77) bei Verwendung einer Elektrokorund- und Siliziumkarbidkörnung und nach DIN 848 (Ausgabe 3/80) bei Verwendung einer Diamantkörnung.

2. Zahnärztliches Handstück nach Anspruch 1, dadurch gekennzeichnet, daß die Trägerschicht (11) aus einem auf eine galvanisierbare Oberfläche des Handstückteils (10) galvanisch aufgebrachten Material gebildet ist.

3. Zahnärztliches Handstück nach Anspruch 1, dadurch gekennzeichnet, daß die Körner (12) in einer Nickelschicht (13) eingebettet sind.

4. Zahnärztliches Handstück nach Anspruch 2, dadurch gekennzeichnet, daß die Nickelschicht (13) von einer Chromschicht von 1 bis 3 $\mu$m überzogen ist.

5. Zahnärztliches Handstück nach einem der Ansprüche 1 bis 4, dadurch gekennzeichnet, daß als Einlagerungen Korundkörner (12) vorgesehen sind.

6. Zahnärztliches Handstück nach einem der Ansprüche 1 bis 3, dadurch gekennzeichnet, daß als Einlagerungen Diamantkörner vorgesehen sind.

## Claims

1. A dental hand-piece, characterised in that at least parts of its surface are provided with a carrier layer (11), into which grains (12) of a hardness greater than 2 000 HV are embedded with an adequate adhesion in such manner that a surface roughness is formed which corresponds to coarsenesses for abrasives on substrates in the region of 240 to 1 200, preferably to a coarseness of grain size 360, in accordance with DIN 69176 (edition 8/77), using an electro-corundum coarseness and silicon carbide coarseness, and in accordance with DIN 848 (edition 3/80) using a diamond coarseness.

2. A dental hand-piece as claimed in Claim 1, characterised in that the carrier layer (11) consists of a material which is electrolytically applied onto an electroplatable surface of the hand-piece part (10).

3. A dental hand-piece as claimed in Claim 1, characterised in that the grains (12) are embedded in a nickel layer (13).

4. A dental hand-piece as claimed in Claim 2, characterised in that the nickel layer (13) is covered with a chromium layer of 1 to 3 $\mu$m.

5. A dental hand-piece as claimed in one of Claims 1 to 4, characterised in that corundum grains (12) are provided as embedded particles.

6. A dental hand-piece as claimed in one of Claims 1 to 3, characterised in that diamond grains are provided as embedded particles.

## Revendications

1. Pièce à main de dentisterie, caractérisée par le fait qu'au moins des parties de sa surface sont munies d'une couche de support (11) dans laquelle des grains (12) possédant une dureté supérieure à 2 000 HV et présentant une adhérence suffisante sont enchâssés de telle sorte que l'on obtient une rugosité de surface qui correspond à des granulations pour des abrasifs sur des supports se situant dans la gamme de 240 à 1 200 et de préférence à une granulation avec une taille de grains de 360, conformément à DIN 69176 (Edition 8/77) dans le cas de l'utilisation d'une granulation du corindon et du carbure de silicium et selon DIN 848 (Edition 3/80) dans le cas de l'utilisation d'une granulation du diamant.

2. Pièce à main de dentisterie suivant la revendication 1, caractérisée par le fait que la couche de support (11) est constituée en un matériau déposé par voie galvanique sur une surface pouvant être galvanisée, de la pièce à main (10).

3. Pièce à main de dentisterie suivant la revendication 1, caractérisée par le fait que les grains (12) sont enchâssés dans une couche de nickel (13).

4. Pièce à main de dentisterie suivant la revendication 1, caractérisée par le fait que la couche de nickel (13) est recouverte par une couche de chrome d'une épaisseur de 1 à 3 $\mu$m.

5. Pièce à main de dentisterie suivant l'une des revendications 1 à 4, caractérisée par le fait qu'il est prévu des grains de corindon (12) en tant qu'éléments d'insertion.

6. Pièce à main de dentisterie suivant l'une des revendications 1 à 3, caractérisée par le fait qu'il est prévu des grains de diamant en tant qu'éléments d'insertion.

FIG1

FIG 2